# EUROPEAN PATENT APPLICATION

(11) **EP 2 338 415 A1**
(43) Date of publication of application: **29.06.2011**
(21) Application number: 09180429.4
(22) Date of filing: 22.12.2009
(51) Int. Cl.: A61B 5/11, A61G 12/00

(54) **Sensor system**

(71) Applicant: Koninklijke Philips Electronics N.V., 5621 BA Eindhoven (NL)
(72) Inventor: Bruekers, Alphons, A.M.L., 5600 AE, Eindhoven (NL); Breebaart, Dirk, J, 5600 AE, Eindhoven (NL); Boughorbel, Sabri, 5600 AE, Eindhoven (NL)
(74) Representative: Damen, Daniel Martijn

(57) **Abstract**

A sensor system (100) is disclosed, comprising a sensor array (110), the sensor array (110) comprising a substrate layer (120, 150) and a plurality of individual pressure sensor elements (130) for measuring a pressure exerted on said sensor array (110) by an object. The sensor elements (130) define a sensor plane (140). A cover member (160) is positionable over at least part of said substrate layer (120, 150), so as to cover said sensor plane (140). Said cover member (160) comprises a support surface (161) for supporting the object and has a dimension (d1) in a direction perpendicular that said support surface (161). The material has a ratio (R) between a Young's modulus (E) of said material and said dimension (d1) that is between 1.10⁴ and 1.10⁷ Pa/m.

## Description

### FIELD OF THE INVENTION

The present invention relates to a sensor system.

### BACKGROUND OF THE INVENTION

Sensor systems are widely known and widely used in an almost unlimited number of applications and application areas. One particular type of sensor system comprises a so-called FLASC or Flexible Large-Area Sensor Carrier. Such a sensor system is for example known from Tekscan, which produces the clinical BPMS^{™} system (Body Pressure Measurement System). Typically such a system comprises flexible polyester sheets on which electrically conductive electrodes are positioned such that the electrodes face each other. The electrodes are arranged in a patterned manner. The known BPMS^{™} system is a pressure sensitive resistive system wherein between the conductive electrodes a coating is provided. When pressure is applied on the coating, its electrical resistance is changed and thus pressure data can be obtained. Different types of pressure sensitive sensor systems are also known, such as capacitive systems and systems using for example optical fibers and photo diodes.

An example of a pressure sensor system is shown in United States patent document US 2009/0070939 in which a system for the prevention of pressure ulcers with patients is disclosed. The system comprises a flat cushioning pad in which an array of pressure transducers is provided. The system further comprises a processor that is arranged to collect the signals that originate from the sensors in the array. The processor executes software that analyses the signals from the sensors and may provide the results to a (computer) display showing a graphical map of the pressure data. The system may be provided with a cover member that is positionable on top of the cushioning pad with the array of sensors and on which the patient lies during use of the system. The cover member is a thin porous cloth attached over a layer of moisture-absorbing material. The material of the cloth is disposable and can be removed from the cushioning pad with the array of sensors.

The known pressure sensitive systems comprising a substantially flat pressure sensitive sensor array have a number of concerns that relate to the way these systems are normally used.

When the sensor system is used in combination with a (soft) mattress such as when used to avoid the occurrence of ulcers in a patient the flat sensor array or sensor mat is often placed on the (soft) mattress. This has the effect that the sensor mat will deform significantly when a person is positioned on the sensor mat. This deformation of the sensor mat results in erroneous signals generated by the sensor mat due to folding and significant deformation. Furthermore, significant deformation (depending on the softness of the mattress) may easily cause the individual sensor elements of the sensor mat to become damaged due to strain.

In practice the number of individual sensor elements (or sensels) is limited. This has the effect that an area exists in the sensor mat that is not covered by the pressure sensitive sensor elements and consequently that for example very small object or objects with an articulated spherical shape where that object touches the sensor mat are not properly detected. Furthermore, such objects give rise to relatively small contact areas and in particular when such objects carry sufficient weight, the local pressure on individual sensor elements may become large. This requires a large dynamic range of the pressure sensor elements, which results in expensive sensor elements to be used. Furthermore, as the number of individual sensor elements is limited, the known sensor systems (or sensor mats) are sensitive to spatial aliasing when changes in the pressure values generated by one or more objects on the sensor mat fluctuate at a spatial rate that lies above half the spatial sampling frequency of the individual sensor elements. This results in erroneous sensor data being generated by the sensor system.

### OBJECT OF THE INVENTION

It would therefore be desirable to provide a sensor system of the abovementioned type that does not suffer from the abovementioned concerns. More in particular it would be desirable to provide a sensor system that exhibits a reduced deformation when used in combination with a (soft) mattress, that has an improved accuracy and lifetime.

### SUMMARY OF THE INVENTION

The inventors have realized that the sensor systems as discussed are in fact spatial sampling devices and the abovementioned concerns can be addressed by providing a physical spatial low-pass filter on an input signal which is formed by the pressure exerted on the sensor array of individual pressure sensor elements. The inventors having realized this, have proposed a sensor system that comprises a sensor array, the sensor array comprising a substrate layer and a plurality of individual pressure sensor elements for measuring a pressure exerted on said sensor array by an object. The sensor elements define a sensor plane. Further, a cover member is provided that is positionable over at least part of said substrate layer, so as to cover said sensor plane, at least partly. The cover member comprises a support surface for supporting the object. The cover member has a dimension in a direction perpendicular that said support surface and comprises a material having a ratio between a Young's modulus of said material and said dimension that is between 1.10⁴ and 1.10⁷ Pa/m.

By providing a cover member that is characterized by said ratio of said dimension, which dimension normally will be its thickness, and the Young's modulus which is a mechanical property of the material the cover member is made of, a mechanical low-pass filter is provided. Such a low-pass filter has the effect that a load or pressure that is exerted by an object that is located on the support surface of the cover member is spread out over a larger area than the actual area of the object that faces the support surface. Hence, the maximum pressure exerted will have a reduced dependency on the actual shape and the position of the object. Furthermore, smaller objects are detectable and spatial aliasing will reduce as spreading of the pressure exerted will have the result that more sensor elements will detect a slowly spatially changing pressure pattern. As an additional advantage the dynamic pressure range of the sensor elements can be reduced, as the spreading of the load will flatten the pressure profile and the maximum pressure value is reduced. As yet another advantage, the influence of temperature on pressure sensors is reduced, because of the thermal isolation properties of the cover member. Alternatively, or additionally, the potentially negative influence of temperature on pressure sensors can be reduced by measuring and compensating for temperature differences.

In an embodiment the ratio is between 1.10⁵ and 1.10⁶ Pa/m, preferably between 2.10⁵ and 1.10⁶ Pa/m. These ranges have proven to give very good results in practice.

In an embodiment the cover member comprises a material chosen from a group of materials having a Young's modulus between 1 kPa and 1 MPa, preferably between 10 kPa and 750 kPa and even more preferably between 50 kPa and 500 kPa. These ranges of Young's modulus are representative of materials that have proven to give very good results in practice.

In an embodiment said material is chosen from a group comprising Tempur, visco-elastic memory foam, fabric cover materials filed with feathers, cotton, synthetic (polystyrene) foams, wool, and latex. Preferably, the material is chosen from a group of materials having a low thermal conductivity. This will reduce the effect of body temperature and temperature differences spatially and temporary on temperature sensors that may be present, when the cover member is used to support a patient for example.

In an embodiment said material has an open structure. This is particularly advantageous when the sensor system is used with people on a bed or the like as the open structure allows moisture to be transported away from the support surface. Furthermore, the open structure of the cover member increases safety of the sensor system in case a person, for example a small child, is lying face-down on the cover member.

In an embodiment the cover member comprises a plurality of layers, which enables fine-tuning of the mechanical properties of the cover member.

In an embodiment said dimension is between 0.5 to 20 cm, preferably between 5 to 15 cm. These dimensions, or thickness of the cover member make it suitable to be used as a mattress to be used with patients for example. Depending on the specific needs, even a thickness of 1 cm can be sufficient.

In an embodiment the pressure sensitive elements are chosen from a group comprising: pressure sensitive resistive elements, pressure sensitive capacitive elements, piëzo-electric elements, piëzo-resistive elements and optical elements. Providing a robust and cost-effective embodiment.

In an embodiment the sensor array is a Flexible Large-Area Sensor Carrier (FLASC), which provides a cost-effective substrate for carrying the sensor array.

In an embodiment the cover member is removably attachable to the sensor array, which ensures that during use the cover member stays attached to the sensor array.

In an embodiment the sensor array further comprises one or more temperature sensitive sensor elements, which makes it possible to compensate for a temperature dependency of the pressure sensor elements. This is in particular advantageous when the sensor system is used with people as people will heat up the sensor array. With the possibility of compensating the temperature dependency of the pressure sensor elements with data generated by the temperature sensor elements a further improved pressure measurement is achieved.

### BRIEF DESCRIPTION OF THE DRAWINGS

Fig. 1 shows an exploded view of an exemplary embodiment of a sensor system;
Fig. 2 shows a schematic view of a pressure sensitive sensor array;
Fig. 3 shows a sensor array with and without a cover member and an object, and
Fig. 4 shows a pressure distribution graph for Figure 3.

### DETAILED DESCRIPTION OF EXAMPLES

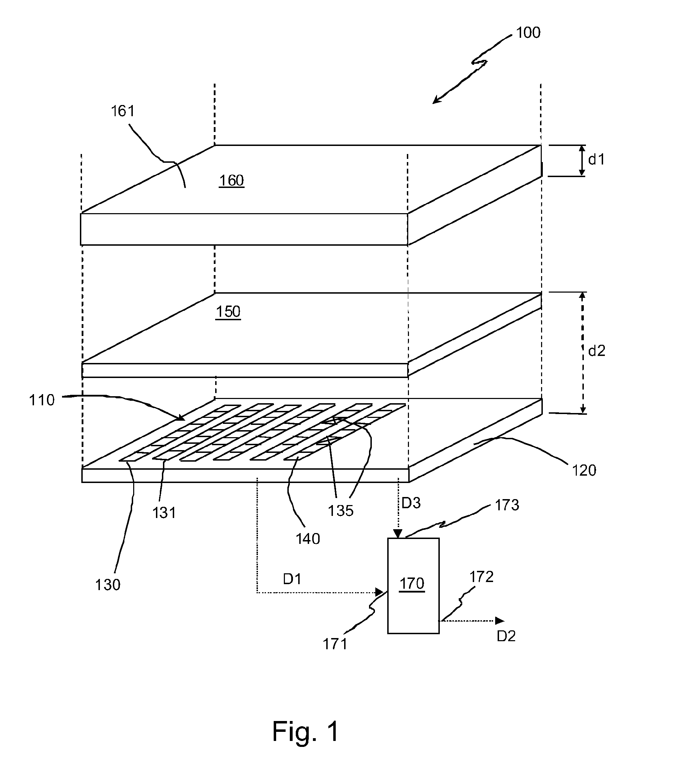

Figure 1 shows a schematic example of a sensor system 100 according to the invention. The sensor system 100 comprises a sensor array 110 which comprises or is provided on a substantially flat first substrate layer 120. The sensor array 110 comprises a plurality of individual pressure sensor elements 130 which sensor elements 130 are arranged to measure pressure exerted on the sensor array by an object (not shown) which may be positioned on a support surface 161. As can be seen, the sensor elements 130 in the example shown define a substantially flat sensor plane 140. The sensor elements 130, in the example of Figure 1, are covered by a second substrate layer 150 of similar dimensions as the first substrate layer 120. The provision of the second substrate layer 150 is not essential, but may be convenient for protecting the sensors elements 130.

The sensor system 100 of Figure 1 further comprises a substantially flat cover member 160 that, in the example of Figure 1, can be placed on top of the second substrate layer 150. However, in case the second substrate layer 150 is not present the cover member 160 may be placed or arranged directly on the sensor array 110 and thus over at least the first substrate layer 120, so as to cover said sensor plane 140.

A control unit 170 may further be provided. The control unit 170 comprises a first input 171 for receiving a first input signal D1 or sensor data generated by the sensor array 130. The control unit 170 may further comprise a first output 172 for providing a first output signal D2 comprising, at least part of, said sensor data. The output signal D2 may for example be supplied to a further control unit, a computer system or the like in which the output signal D2 is further manipulated to be suitable for display purposes on a display device for example.

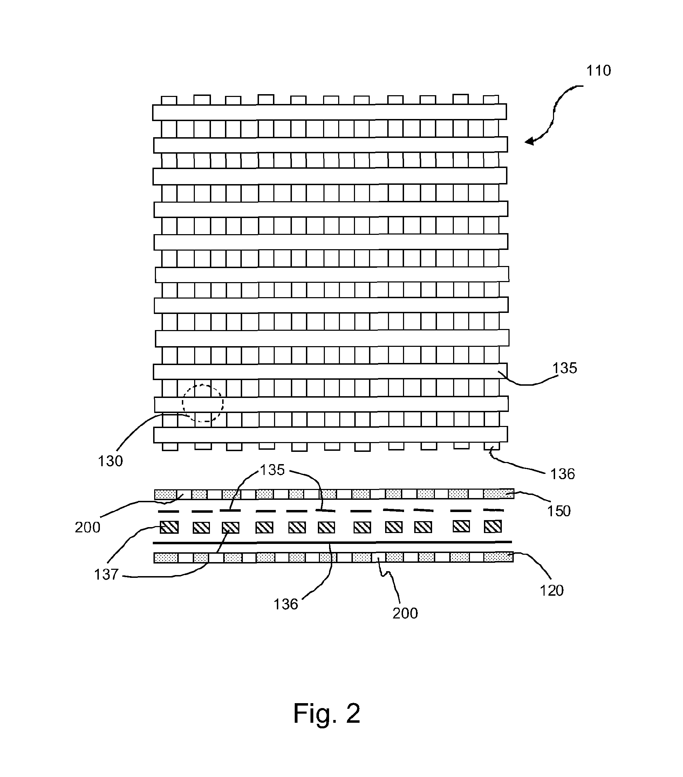

Figure 2 (upper half) shows a schematic example of a sensor array 110 in plan view, comprising a plurality of first electrodes 135 that are attached to the second substrate layer 150 and a plurality of second electrodes 136 that are attached to the first substrate layer 120. As can be seen in Figure 2, the first electrodes 135 and the second electrodes are orthogonal with respect to each other. However, other patterns are also conceivable and the present invention is not limited to the example shown in Figure 2. The first and second electrodes 135, 136 are electrically conductive electrodes. Between the first electrodes and the second electrodes at those locations where said electrodes cross, pressure sensitive resistive material 137 is provided. This is shown in the lower half of Figure 2, showing a schematic cross-sectional view. The pressure resistive material 137 prevents direct contact between the first and second electrodes 135, 136. The pressure resistive material 137 is for example a thin semi-conductive coating (ink), which material 137 has a varying electrical resistance when it is subjected to a varying pressure. The sensor array 110 hence comprises a plurality of pressure sensor elements 130 (indicated in Figure 2 with a dotted circle) that are formed at each intersection of a first electrode 135, a second electrode 136. The sensor array 110 is arranged to measure changes in current flow between the electrodes 135, 136 at each intersection when for example an object is placed on the second substrate layer 150. The measured changes in current flow (resulting from a change in the resistance of the pressure resistive material) represent the sensor data D1 (see Figure 1).

In the example of Figure 2, the sensor system 100 utilizes pressure sensitive resistive material to form pressure sensitive sensor elements. Other solutions are also known, such as pressure sensitive capacitive sensor elements wherein the electrodes are separated by an air gap and the distance between the electrodes is a measure of the pressure exerted. Other solutions involve the use of piëzo-electric elements, or optical elements. Such alternative solutions are known per se and to the person skilled in the art. An example of a so-called FLASC or Flexible Large-Area Sensor Carrier as schematically shown in Figure 1 and 2 is the Tekscan BPMS^{™} system. The BPMS^{™} system may for example used for monitoring patients in bed to avoid the occurrence of ulcers.

The cover member 160 has a dimension d1 or thickness in a direction that is orthogonal with respect to the support surface 161. Preferably the thickness d1 of the cover member 160 is larger, more preferably substantially larger than a corresponding dimension or thickness d2 of the sensor array 110 respectively the sensor array 110 and both substrates 120, 150. This is schematically indicated in Figure 1 with a dotted line for d2, as Figure 1 is an exploded view. The cover member 160 is made of a relatively soft material which makes is suitable for example to be used as a mattress. Examples of suitable materials are foams, fiberfills or natural soft materials. Preferably the material of the cover member 160 has an open structure that allows moisture to be transported away from the support surface 161 of the cover member 160. This is not shown in detail in the drawing, but it will be clear to a person skilled in the art how to achieve such an open structure. It is also possible to provide the sensor array 110 and the corresponding substrates 120, 150 with an open structure. This has schematically been indicated in Figure 2 by means of through holes 200. The open structure of the sensor array 110 and respective substrates 120, 150 allows moisture to be transported away from the sensor array 110 and allows a person that is lying face-down on the sensor array 110 to breathe, thus increasing safety.

As will be explained in more detail below, the cover member 160 when positioned on and over the sensor array 110, will act as a mechanical low-pass filter for pressure exerted, i.e. a pressure signal applied to the sensor array 110. The inventors have realized that the sensor system 100 is in fact spatial sampling device as the individual sensor elements 130 are distributed in a patterned manner over the sensor array 110. The cover member 160 comprises or is made of a material that exhibits a ratio between the Young's modulus E thereof and the thickness d1 that is between 1.10⁴ and 1.10⁷ Pa/m, preferably between 1.10⁵ and 1.10⁶ Pa/m and even more preferably between 2.10⁵ and 1.10⁶ Pa/m. These ranges have proven to give very good results in practice.

By providing a cover member 160 that exhibits said ratio R, i.e. E/d1, a mechanical low-pass filter is provided. Such a low-pass filter has the effect that a load or pressure that is exerted by an object that is located on the support surface 161 of the cover member 160 is spread out over a larger area than the actual area of the object that faces the support surface 161. Hence, the maximum pressure exerted will have a reduced dependency on the actual shape of the object. Furthermore, smaller objects are detectable and spatial aliasing will reduce as spreading of the pressure exerted will have the result that more sensor elements will detect a changing pressure pattern. As an additional advantage the dynamic pressure range of the sensor elements can be reduced, as the spreading of the load will flatten the pressure profile and the maximum pressure value is reduced. As yet another advantage, the preferably low thermal conductivity of the cover member 160 results in a decrease in temperature changes of the pressure sensors, both as a function of place and time.

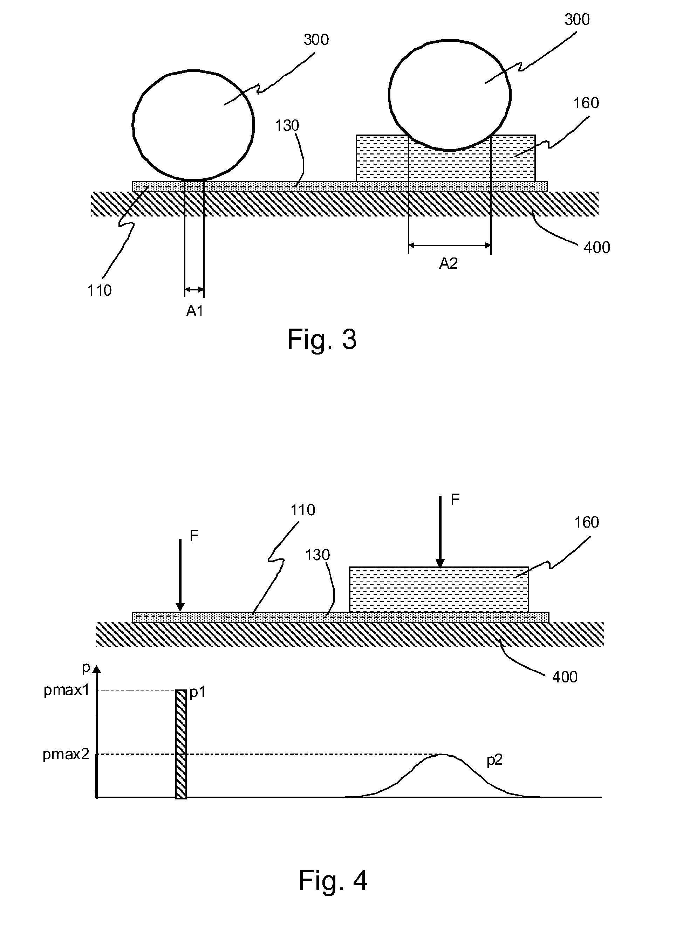

These advantages are illustrated in Figures 3 and 4 respectively.

Figure 3 shows in the left-hand part thereof an object 300 that is positioned directly on the sensor array 110 or FLASC, i.e. the sensor array 110 together with both substrates 120, 150. The sensor array 110 is in turn placed on a hard surface 400. In the right-hand part of Figure 3 it is illustrated that the same object 300 (same size, shape and weight) is now placed on a cover member 160 which is in turn positioned on the sensor array 110. As can be seen, the object 300 has a contact area A1 when it is directly positioned on the sensor array 110, but has a much larger contact area A2 when positioned on the cover member 160 according to the present invention. This means that the pressure exerted on the sensor array 110 is spread over a larger contact area (which may be even larger than contact area A2 due to the fact that the material of the cover member 160 resists to shear forces) than the contact area A1, which means that locally, i.e. per pressure sensor element, the pressure is lowered. Hence, the dynamic pressure range of the individual pressure sensor elements can be reduced and thus cheaper sensor elements can be used and/or sensor elements with a larger sensitivity in a smaller pressure range can be applied. Furthermore, this may contribute to a longer lifespan of the pressure sensor elements 130.

This is further illustrated in Figure 4, where the pressure distribution in case of a point-shaped load on a hard surface is compared with the same load on a soft surface. Figure 4 is a schematic drawing only and it should be noted that in practice the surface below both pressure distribution lines p1 and p2 is the same, as the force F exerted is the same in both instances. Furthermore, in practice the pressure distribution line p1 is not rectangular of course, but will also have a Gauss-like distribution. It will be clear for a person skilled in the art that Figure 4 is illustrative only. This results in a lower maximum value of the pressure pmax2 than the maximum value pmax1 in the case of a point load. This shows the effect of the cover member 160.

From the above examples as shown in Figure 3 and 4 it can also be seen that the sensor array 110, when the cover member 160 according to the present invention is used, be able to detect smaller objects. The larger contact area A2 will encompass more pressure sensor elements 130 than the smaller contact area A1. Hence, as more individual sensor elements 130 take part in the detection of a pressure signal, the better the sensitivity will be. Furthermore, potential measurement errors or measurement inaccuracies are distributed randomly across different sensors. By combining these values across multiple sensors, the effective accuracy is increased. Similarly, the effect of spatial aliasing will also be reduced. This is because the change in pressure along the different pressure sensors will be more smooth, as indicated in the right-hand side of Figure 4. The cover member operates as a mechanical spatial low-pass filter that removes (or attenuates) high spatial frequencies from the pressure pattern that is excerted onto sensor array 110. The various sensors, on the other hand, are separated by a certain distance. From digital sampling theory, it is well known that in order to avoid aliasing during sampling, the distance between sensors is upper bounded by the highest spatial frequency present in the exerted pressure pattern. Therefore, if the high spatial frequencies in the pressure pattern are reduced by the cover member, the distance between sensors can be increased, and hence the number of sensors is decreased, resulting in a decreased cost.

Furthermore, given a certain distance between sensors and the absence of the cover member 160, there is a risk that the force F as depicted in Figure 4 may result in a very small contact area that falls just in-between pressure sensors. This is highly undesirable because the force F will in such case not be detectable. The application of the cover member 160 will result in (1) a larger contact area A2, and (2) a larger pressure area as indicated in the right-hand side of Figure 4, hence alleviating the risk of forces that are not detected.

To further improve the sensor system 100 according to the present invention, an embodiment thereof may comprise a sensor array 110 which further comprises one or more temperature sensitive sensor elements 135. This is shown in Figure 1. The provision of such temperature sensor elements 135 makes it possible to compensate for a temperature dependency of the pressure sensor elements 130. This is in particular advantageous when the sensor system 100 is used with people as people will heat up the sensor array 110. With the possibility of compensating the temperature dependency of the pressure sensor elements 130 with data generated by the temperature sensor elements 135 a further improved pressure measurement is achieved. This is indicated in Figure 1 with an input signal D3 that is supplied to an input 173 of the control unit 170. The control unit 170 may in such case be arranged to correct the output signal D2 using the input signal D3.

It is noted that although the cover member 160 is heretofore shown as a single piece of material, the cover member 160 is not limited thereto. The cover member may also comprise several layers of material each layer having specific mechanical properties.

In the above description the cover member and sensor array were shown to be of a generally rectangular shape. It is to be understood however that in principle any shape of the cover member and the sensor array are conceivable. Furthermore, both the cover member and the sensor array maybe provide with means to attach the cover member to the sensor array. Such means may include one or more of buttons, Velcro strips, ropes, zippers etcetera.

In the above description the invention has been described by means of the example of a substantially flat cover member and a substantially flat sensor array. It is to be understood however that the invention is not limited to flat objects only. It is very well possible to extend the application of the invention to non-flat objects also. For example it is possible to provide a non-flat object that is provided with an array of sensors whereby the non-flat array of sensors can be covered by a cover member that, at least partly, envelops the non-flat sensor array. Furthermore, it is also possible that the cover member is in fact larger than the sensor array it is intended to (at least partly) cover. This means that the cover member may for example very well be draped around the edges of the sensor array.

While the subject-matter has been illustrated in the drawings and the foregoing description, such illustration and description are to be considered illustrative or exemplary and not restrictive; the subject-matter is not limited to the disclosed embodiments. Other variations to the disclosed embodiments can be understood and effected by those skilled in the art of practicing the claimed subject-matter, from a study of the drawings, the disclosure and the appended claims. Use of the verb "comprise" and its conjugations does not exclude the presence of other elements other than those stated in a claim or in the description. Use of the indefinite article "a" or "an" preceding an element or step does not exclude the presence of a plurality of such elements or steps. The Figures and description are to be regarded as illustrative only and do not limit the subject-matter. Any reference sign in the claims should not be construed as limiting the scope.

## Claims

1. A sensor system (100), comprising:
a sensor array (110), the sensor array (110) comprising a substrate layer (120, 150) and a plurality of individual pressure sensor elements (130) for measuring a pressure exerted on said sensor array (110) by an object, which sensor elements (130) define a sensor plane (140), and
a cover member (160) positionable over, at least part of, said substrate layer (120, 150), so as to cover, at least part of, said sensor plane (140), wherein said cover member (160) comprises a support surface (161) for supporting the object,
**characterized in that**
the cover member (160) has a dimension (d1) in a direction substantially perpendicular that said support surface (161) and comprises a material having a ratio (R) between a Young's modulus (E) of said material and said dimension (d1) that is between 1.10⁴ and 1.10⁷ Pa/m.

2. Sensor system (100) according to claim 1, wherein the ratio (R) is between 1.10⁵ and 1.10⁶ Pa/m.

3. Sensor system (100) according to claim 1 or 2, wherein the ratio (R) is between 2.10⁵ and 1.10⁶ Pa/m.

4. Sensor system (100) according to any of the previous claims, wherein the cover member (160) comprises a material chosen from a group of materials having a Young's modulus (E) between 1 kPa and 1 MPa.

5. Sensor system (100) according to any of the previous claims, wherein the cover member (160) comprises a material chosen from a group of materials having a Young's s modulus (E) between 10 kPa and 750 kPa.

6. Sensor system (100) according to any of the previous claims, wherein the cover member (160) comprises a material chosen from a group of materials having a Young's modulus (E) between 50 kPa and 500 kPa.

7. Sensor system (100) according to any of the previous claims, wherein said material is chosen from a group comprising: Tempur, visco-elastic memory foam, fabric cover materials filed with feathers, cotton, synthetic (polystyrene) foams, wool, and latex, preferably the material is chosen from a group of materials having a low thermal conductivity.

8. Sensor system (100) according to any of the previous claims, wherein said material has an open structure.

9. Sensor system (100) according to any of the previous claims, wherein the cover member (160) comprises a plurality of layers.

10. Sensor system (100) according to any of the previous claims, wherein said dimension (d1) is between 0.5 to 20 cm, preferably between 5 to 15 cm.

11. Sensor system (100) according to any of the previous claims, wherein the sensor array (110) further comprises one or more temperature sensitive sensor elements (135).

12. Sensor system (100) according to any of the previous claims, wherein the pressure sensitive elements (131) are chosen from a group comprising: pressure sensitive resistive elements, pressure sensitive capacitive elements, piëzo-electric elements, piëzo-resistive elements and optical elements.

13. Sensor system (100) according to any of the previous claims, wherein the sensor array (110) is a Flexible Large-Area Sensor Carrier (FLASC).

14. Sensor system (100) according to any of the previous claims, wherein the cover member (160) is removably attachable to the sensor array (110).
